# DEMANDE DE BREVET EUROPEEN

(11) **EP 4 692 718 A1**
(43) Date de publication de la demande: **11.02.2026**
(21) Numéro de dépôt: 25179562.1
(22) Date de dépôt: 28.05.2025
(51) Int. Cl.: G01B 5/00, G06G 1/00, A61B 5/00, A61G 12/00, G16H 10/20, G06F 3/00

(54) **DISPOSITIF D'AIDE AU RÉGLAGE D'UN VENTILATEUR MÉDICAL**

(30) Priorité: 07.08.2024 FR 2408742
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: DELANGHE, Arnaud, 92220 Bagneux (FR)
(74) Mandataire: Air Liquide

(57) **Abrégé**

L'invention concerne un dispositif (1) d'aide au réglage d'un ventilateur médical servant à fournir un gaz respiratoire à un patient comprenant un corps principal (2) avec deux surfaces (3, 4) opposées, une première série d'emplacements (10) avec des emplacements (11) comprenant une indication (12) relative au ressenti du patient par rapport à une ventilation lui ayant été appliquée, et une seconde série d'emplacements (20) avec des emplacements (21) comprenant une indication de réglage du ventilateur (22). Des éléments indicateurs (13, 23) sont mobiles relativement aux emplacements (11) de sorte qu'un positionnement du premier élément indicateur (13) face à l'un des emplacements (11) de la première série d'emplacements (10) engendre un positionnement simultané du second élément indicateur (23) face à l'un des emplacements (21) de la seconde série (20) d'emplacements (20).

## Description

L'invention concerne un dispositif ou outil d'aide au réglage d'un ventilateur médical délivrant un gaz respiratoire à un individu en ayant besoin, c'est-à-dire un patient.

La ventilation mécanique est un traitement respiratoire indiqué pour des patients insuffisants respiratoires. Elle s'opère à l'aide d'un appareil de ventilation ou ventilateur médical délivrant un gaz respiratoire au patient, tel de l'air ou un mélange air/oxygène, selon un mode de ventilation non-invasive (VNI) lorsqu'on a recours à une interface-patient de type masque respiratoire ou analogue, ou selon un mode de ventilation invasive (VI) lorsque l'interface est une canule de trachéotomie ou analogue.

Le réglage des ventilateurs médicaux est réalisé au travers d'environ une dizaine paramètres ventilatoires ajustables qui dépendent à la fois de la pathologie du patient et de son état respiratoire du moment, mais aussi de la tolérance et du ressenti du patient pendant son traitement.

Ainsi, pour affiner les réglages et assurer un traitement efficace et bien toléré, une bonne communication entre le praticien et son patient est indispensable.

Or, cette communication est rendue difficile en cours de traitement, voire impossible, du fait de l'interface (i.e. masque ou canule) qui équipe le patient et des pressions générées dans ses voies aériennes par le ventilateur.

On connait les documents suivants :
- Münevver Otuzoglu et al., Determining the effectiveness of illustrated communication material for communication with intubated patients at an intensive care unit, Int. J. of Nursing Practice; vol. 5, n°5, 30 Sept. 2013, p. 490-498,
- EP4406575 enseigne un moyen de connaître des patients dont les poumons répondent favorablement à une augmentation de pression expiratoire positive (PEP).
- US2003/019115 propose un dispositif de type réglette à curseur mobile servant à déterminer le taux de filtration glomérulaire en tant que marqueur ou fonction rénale chez un patient.

L'invention vise à résoudre ce problème en proposant un dispositif d'aide au réglage permettant de déterminer facilement et rapidement les paramètres **ventilatoires** les mieux adaptés à un patient donné, même lorsque le patient est équipé d'une interface respiratoire l'empêchant de l'exprimer.

Une solution de l'invention concerne un dispositif d'aide au réglage d'un ventilateur médical servant à fournir un gaz respiratoire à un patient comprenant :
- un corps principal comprenant une première surface et une seconde surface opposée à la première surface,
- une première série d'emplacements comprenant plusieurs emplacements juxtaposés les uns aux autres, c'est-à-dire une pluralité de « premiers emplacements », ladite première série d'emplacements étant agencée sur la première surface, chaque emplacement comprenant une indication relative au ressenti du patient par rapport à une ventilation lui ayant été appliquée,
- une seconde série d'emplacements comprenant plusieurs emplacements juxtaposés les uns aux autres, c'est-à-dire une pluralité de « seconds emplacements », ladite seconde série d'emplacements étant agencée sur la première surface ou la seconde surface, chaque emplacement comprenant au moins une indication de réglage du ventilateur,
- un premier élément indicateur agencé en regard de la première série d'emplacements et mobile relativement aux emplacements de la première série d'emplacements, et
- un second élément indicateur agencé en regard de la seconde série d'emplacements et mobile relativement aux emplacements de la seconde série d'emplacements.

De plus, le premier élément indicateur coopère avec le second élément indicateur de sorte qu'un positionnement par l'utilisateur du premier élément indicateur face à l'un des emplacements de la première série d'emplacements engendre un déplacement simultané du second élément indicateur et un positionnement dudit second élément indicateur face à l'un des emplacements de la seconde série d'emplacements.

Selon le mode de réalisation considéré, le dispositif d'aide au réglage de l'invention peut comprendre l'une ou plusieurs des caractéristiques suivantes :
- la première surface constitue une face supérieure du corps principal.
- la seconde surface constitue une face inférieure du corps principal.
- la première surface et la seconde surface sont parallèles l'une à l'autre, c'est-à-dire qu'elles forment des plans parallèles.
- la seconde série d'emplacements est agencée sur la seconde surface et le second élément indicateur agencé mobile en regard de la seconde surface.
- le premier élément indicateur et le second élément indicateur sont agencés sur un élément-curseur commun mobile.
- le premier élément indicateur et le second élément indicateur sont couplés l'un à l'autre.
- l'élément-curseur comprend un manchon agencé coulissant autour du corps principal, c'est-à-dire mobile en translation.
- le manchon peut être transparent et porter les premier et second éléments indicateurs.
- le corps principal comprend des butées d'extrémités pour stopper tout coulissement (i.e. translation) du manchon, c'est-à-dire empêcher qu'il se détache ou se désolidarise du corps principal.
- le corps principal a une forme rectangulaire allongée et les butées d'extrémités sont agencées aux deux extrémités opposées de ladite forme rectangulaire allongée.
- le corps principal a une forme plane.
- le corps principal a une forme allongée.
- le corps principal a une forme rectangulaire ou carrée ou, alternativement, le corps principal a une forme de (demi-)disque ou autre.
- le corps principal est rectangulaire et a une longueur d'environ 20 cm et une largeur d'environ 10 cm.
- le corps principal est rigide ou (légèrement) flexible.
- la première série d'emplacements comprend des emplacements agencés en une première rangée, de préférence une rangée linéaire ou courbe.
- la seconde série d'emplacements comprend des emplacements agencés en une seconde rangée, de préférence une rangée linéaire ou courbe.
- le corps principal constitue un support ou analogue portant les première et seconde séries d'emplacements et les premier et second éléments indicateurs.
- le corps principal est en carton, papier ou polymère, ou leurs combinaison.
- le premier élément indicateur et le second élément indicateur comprennent un marquage ou repère, tel un trait, une flèche ou analogue, ou une fenêtre de lecture ou autre.
- chaque emplacement comprend une indication relative au ressenti du patient par rapport à la pression délivrée par le ventilateur et qui lui est appliquée, par exemple pression trop forte, trop faible ou bien adaptée, ou autres.
- chaque emplacement comprend au moins une indication de réglage du ventilateur relative à la pression appliquée au patient, par exemple augmenter ou diminuer (de quelques mbar) ou maintenir le niveau de pression appliqué.
- chaque emplacement est matérialisé par une case ou analogue.
- le premier élément indicateur et le second élément indicateur sont mobiles simultanément en translation ou en pivotement, de préférence en translation.
- le premier élément indicateur est porté par un curseur translatif guidé par un (ou des) rail(s), glissière(s) ou analogue agencée(s) sur le corps principal du dispositif.
- le second élément indicateur est couplé au curseur portant le premier élément indicateur.
- les emplacements de la première série d'emplacements comprennent chacun une indication relative au ressenti du patient par rapport à la pression du gaz respiratoire, à la résistance ressentie à l'expiration, à la durée d'insufflation de gaz, à la vitesse d'entrée de gaz dans les poumons et/ou à la détection (trigger) du début d'inspiration du patient, en particulier relative à un niveau de ressenti insuffisant, adéquat ou excessif, ou autres.
- les emplacement de la seconde série d'emplacements comprennent une indication de réglage de la pression gazeux à fournir par le ventilateur, de la pression expiratoire, de la durée d'insufflation de gaz, de la pente de la vitesse de fourniture de gaz et/ou de la sensibilité de la détection inspiratoire, en particulier des indications visant à augmenter, maintenir ou diminuer le réglage du (des) paramètre considéré.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un dispositif d'aide au réglage d'un ventilateur médical selon l'invention.
Fig. 2 est un mode de réalisation de la première surface ou face supérieure du dispositif de Fig. 1.
Fig. 3 est un mode de réalisation de la seconde surface ou face inférieure du dispositif de Fig. 1.
Fig. 4 schématise un autre mode de réalisation possible d'un dispositif d'aide au réglage d'un ventilateur médical selon l'invention.
Fig. 5 schématise encore un autre mode de réalisation possible d'un dispositif d'aide au réglage d'un ventilateur médical selon l'invention.
Fig. 6 schématise des paramètres respiratoires pouvant être adaptés ou réglés grâce à un ou des dispositifs d'aide au réglage d'un ventilateur médical selon l'invention.

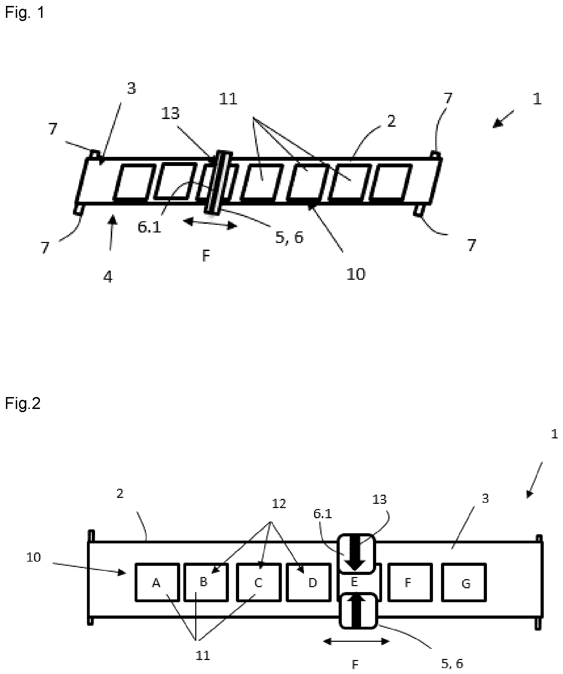

Fig. 1 schématise un mode de réalisation d'un dispositif 1 selon l'invention d'aide au réglage d'un ventilateur médical utilisé pour fournir un gaz respiratoire à un patient en ayant besoin dans le cadre de son traitement, comme de l'air, un mélange air/O₂ ou un autre gaz.

Ce dispositif d'aide au réglage comprend un corps principal 2 ici de forme allongée, rectangulaire et plate présentant une première surface 3, aussi appelée surface supérieure, et une seconde surface 4, aussi appelée surface inférieure, opposée à la première surface 3. Les deux surfaces 3, 4 sont planes et sensiblement parallèles l'une à l'autre, i.e. leurs plans sont parallèles.

Le corps principal 2 forme un support ou analogue. Il peut être constitué de papier, de carton, de polymère ou de tout autre matériau adapté.

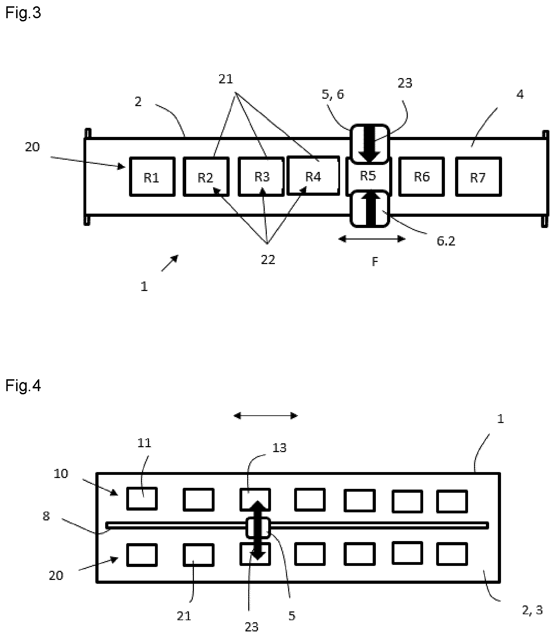

Comme on le voit sur Fig. 1 et Fig. 2, le corps principal 2 porte une première série d'emplacements 10 agencée sur la première surface 3, c'est-à-dire sur la face supérieure du corps 1, et par ailleurs, comme visible sur Fig. 3, une seconde série d'emplacements 20 agencée ici sur la seconde surface 4, c'est-à-dire sur la face inférieure du corps 1.

Toutefois, selon un autre mode de réalisation (non montré), les deux séries d'emplacements 10, 20 pourraient être agencées sur une seule et même surface, typiquement sur la première surface 3 du corps 1.

La première série d'emplacements 10 comprenant plusieurs emplacements 11 juxtaposés les uns aux autres, typiquement agencés en une première rangée d'emplacements 11.

Chaque emplacement 11 comprenant une indication 12, i.e. marquage ou autre, relative au ressenti du patient par rapport à une ventilation lui ayant été appliquée, en particulier par rapport au niveau de pression de ventilation qui lui est appliqué. Les différentes indications 12 sont symbolisées ici par les lettres A à G.

Par exemple, les différentes indications 12 de ressenti (lettres A à G) destinées au patient et lui servant à évaluer son traitement peuvent être du type :
- A : pression bien trop faible, ne m'aide pas du tout.
- B : pression un peu trop faible, plus de pression serait souhaitable.
- C : une pression légèrement supérieure serait idéale.
- D : tout est parfait.
- E : le traitement se déroule bien mais la pression est légèrement trop forte.
- F : pression un peu trop élevée, moins de pression serait souhaitable.
- G : pression bien trop forte, traitement difficile à supporter.

De manière analogue, la seconde série d'emplacements 20 comprenant plusieurs emplacements 21 juxtaposés les uns aux autres, typiquement agencés en une seconde rangée d'emplacements 21.

Chaque emplacement 21 comprenant au moins une indication de réglage du ventilateur 22 qui est préconisée pour chacune des indications 12 de ressenti du patient. Les différentes indications de réglage (R) du ventilateur 22 sont symbolisées ici par R1 à R7.

Par exemple, les différentes indications de réglage (R) du ventilateur 22, en particulier de la pression inspiratoire appliquée au patient, peuvent être du type :
- R1 : augmenter la pression de 3 ou 4 mbar
- R2 : augmenter la pression inspiratoire de 2 ou 3 mbar
- R3 : augmenter la pression de 1 ou 2 mbar
- R4 : maintenir la pression
- R5 : diminuer la pression de -1 ou -2 mbar
- R6 : diminuer la pression de -2 ou -3 mbar
- R7 : diminuer la pression de -3 ou -4 mbar

Bien entendu, les indications 12 de ressenti du patient et les indications de réglage (R) du ventilateur 22 peuvent porter sur d'autres aspects et paramètres respiratoires comme par exemple :
- pour les indications 12 de ressenti : la résistance ressentie à l'expiration, la durée d'insufflation de gaz, la vitesse d'entrée de gaz dans ses poumons, la détection (trigger) du début d'inspiration du patient...
- pour les indications de réglage (R) du ventilateur 22 : la pression d'expiration à appliquer, la durée d'insufflation à régler, la vitesse à modifier, la sensibilité détection à ajuster...

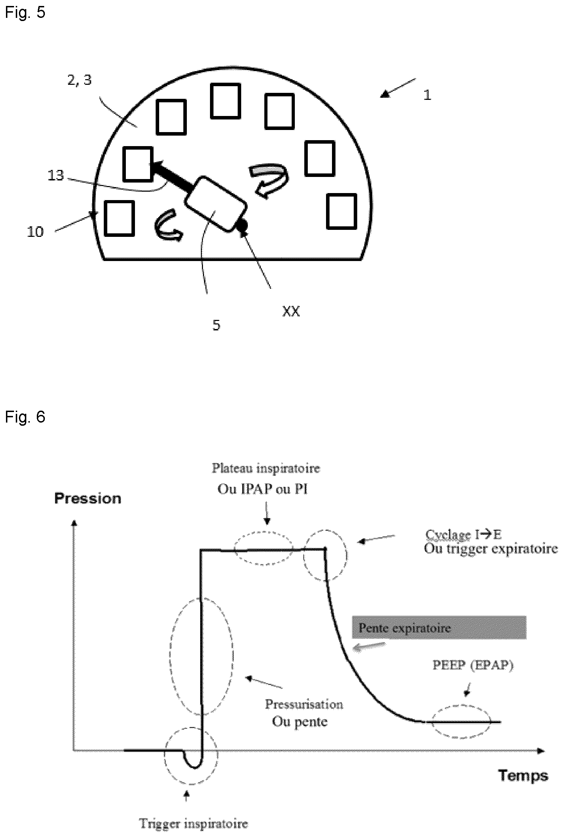

Plus précisément, sur Fig. 6, sont schématisés des paramètres respiratoires pouvant être adaptés ou réglés dans le cadre de l'invention en fonction du ressenti du patient pour ces différents paramètres de réglage qui sont fournis au médecin grâce à un ou des dispositifs d'aide 1 selon l'invention :
- Pression inspiratoire (IPAP ; PI) : pression positive délivrée par le ventilateur durant une inspiration. Elle est typiquement comprise entre 4 et 20 cmH20.
- Pression expiratoire (EPAP ; PEEP) : pression positive maintenue par le ventilateur à la fin de l'expiration du patient. Elle permet d'éviter le collapsus des voies aériennes supérieures à l'expiration, la ré-inhalation de CO₂ avec les masques à fuite intentionnelle, recruter des territoires alvéolaires collapsés, lutter contre la PEP intrinsèque chez les patients atteints de BPCO et/ou améliorer la phonation chez les patients trachéotomisés. Elle est typiquement comprise entre 4 et 15 cmH20.
- Pente inspiratoire (Pressurisation ; Rise time) : temps (en milliseconde) que le ventilateur va mettre pour atteindre la pression inspiratoire établie pour chaque cycle respiratoire. Elle est typiquement comprise entre 0 ms et 1000 ms.
- Déclenchement inspiratoire (trigger inspiratoire) : détection par le ventilateur d'un début d'inspiration du patient et déclencher un cycle ventilatoire. Il peut être déclenché après une variation du débit inspiratoire ou de la pression inspiratoire, ou autre. Sa sensibilité est variable, typiquement entre très sensible et très peu sensible. Elle est typiquement comprise entre 0% et 75% du débit maximal.
- Cyclage (trigger expiratoire) : moment où le ventilateur comprend qu'il y a eu un changement entre inspiration et expiration pour le patient. La détection du passage à l'expiration se fait par détection d'une chute du débit gazeux par rapport au débit de pointe (i.e. pic de débit). Sa sensibilité est variable, typiquement entre très sensible et très peu sensible.
- Rampe expiratoire : durée (en millisecondes) mise par le ventilateur pour atteindre la EPAP.
- Fréquence respiratoire de "sécurité" ou "minimale": il s'agit du nombre de cycles ventilatoires produits par le ventilateur en 1 mn sachant que le malade a la possibilité dans certains réglages d'avoir une fréquence supérieure spontanée s'il le souhaite. Elle est typiquement comprise 5 et 30 cycles/min.

Selon le mode de réalisation, les première et seconde rangées d'emplacements 11, 21 peuvent être linéaires, courbes (arc de cercle) ou autre.

Par ailleurs, un premier élément indicateur 13 est agencé en regard de la première série d'emplacements 10 en étant mobile relativement aux emplacements 11 de la première série d'emplacements 10. Typiquement, le premier élément indicateur 13 est mobile en translation (flèche F) le long de la première série d'emplacements 10 de manière à pouvoir venir se positionner en regard et désigner ainsi l'un ou l'autre des emplacements 11 de la première série d'emplacements 10, en particulier lorsqu'il est déplacé par un utilisateur, i.e. un patient.

De manière analogue, un second élément indicateur 23 est agencé en regard de la seconde série d'emplacements 20 et mobile relativement aux emplacements 21 de la seconde série 20 d'emplacements 21, de manière à pouvoir venir se positionner en regard et désigner ainsi l'un ou l'autre des emplacements 21 de la première série d'emplacements 20.

De plus, selon l'invention, le premier élément indicateur 13 coopère avec le second élément indicateur 23 de sorte qu'un positionnement par l'utilisateur du premier élément indicateur 13 face à l'un des emplacements 11 de la première série d'emplacements 10 engendre un déplacement simultané du second élément indicateur 23 et son positionnement face à l'un des emplacements 21 de la seconde série 20 d'emplacements 21.

Pour ce faire, le premier élément indicateur 13 et le second élément indicateur 23 sont couplés l'un à l'autre.

Avantageusement, ils peuvent être agencés ou portés par un élément-curseur 5 commun mobile, c'est-à-dire déplaçable en translation par exemple.

Cet élément-curseur 5 peut être un manchon 6 agencé autour du corps principal 2 et coulissant le long de ce corps principal 2, c'est-à-dire déplaçable par l'utilisateur de droite à gauche, et inversement, (flèche F) comme schématisé sur les Fig. 1 à Fig. 3.

Dans ce cas, le manchon 6 comprend un côté supérieur 6.1 portant le premier élément indicateur 13 et un côté inférieur 6.2 portant le second élément indicateur 23, par exemple des éléments indicateurs 13, 23 en forme de flèches ou analogue.

Afin d'éviter que l'élément-curseur 5, typiquement le manchon 6, ne puisse coulisser au-delà des extrémités du corps principal 2 et s'en désolidariser, on prévoit préférentiellement des butées d'arrêt 7 ou analogue, à ses deux extrémités comme illustré sur Fig. 1 à Fig. 3.

En utilisation, un utilisateur, typiquement un patient, déplace l'élément-curseur 5, typiquement le manchon coulissant 6, en translation le long de la première série d'emplacements 10 pour positionner le premier élément indicateur 13 face à l'un des emplacements 11 de la première série d'emplacements 10, par exemple face à l'emplacement E, comme illustré en Fig. 2, pour indiquer à son médecin ou analogue que le traitement se déroule bien mais que la pression appliquée est légèrement trop forte.

Le second élément indicateur 23 qui est solidaire du premier élément indicateur 13 étant donné qu'ils sont portés tous les deux par le même manchon coulissant 6, s'est déplacé simultanément et est venu se positionner face à l'un des emplacements 21 de la seconde série d'emplacements 20, qui indique au médecin quel réglage du ventilateur opérer pour répondre à cette pression légèrement trop forte, à savoir le repère R5 illustré en Fig. 3 qui lui préconise de réduire le réglage de pression fournie par le ventilateur de -1 ou -2 mbar.

Le médecin n'a alors plus qu'à appliquer ce réglage pour améliorer le ressenti du patient, donc le traitement proprement dit.

Avantageusement, le médecin peut proposer plusieurs dispositifs 1 d'aide au réglage différents au patient, c'est-à-dire dédiés chacun à un ressenti patient différent et aux réglages (R) correspondants, par exemple l'un portant sur la pression d'insufflation, un autre sur la résistance ressentie à l'expiration, un autre sur la durée d'insufflation de gaz, un autre sur la vitesse d'entrée de gaz dans ses poumons et un dernier sur la détection (trigger) du début d'inspiration du patient... Ainsi, le médecin pourra affiner le traitement en opérant plusieurs modifications de réglages sur le ventilateur en fonction des ressentis du patient et des réglages correspondants proposés par chaque dispositif 1 d'aide au réglage.

Fig. 4 schématise un autre mode de réalisation possible du dispositif 1 d'aide au réglage d'un ventilateur médical selon l'invention, lequel est similaire à celui de Fig. 1, mais dans lequel les première et seconde séries d'emplacements 10, 20, i.e. les première et seconde rangées d'emplacements 11, 21, sont agencés sur une même surface du corps principal 2, à savoir ici la première surface 3.

Dans ce cas, l'élément-curseur 5 peut être aussi un manchon coulissant 6, par exemple transparent portant les deux éléments-indicateurs 13, 23 (i.e. flèches ici), comme sur Fig. 1 mais peut avoir aussi une forme différente de celle d'un manchon 6, comme par exemple il peut être un curseur translatif guidé par un ou (des) rail 8, glissière(s) ou analogue agencée(s) sur le corps principal 2 du dispositif 1, comme schématisé en Fig. 4.

Fig. 5 schématise encore un autre mode de réalisation possible d'un dispositif 1 d'aide au réglage d'un ventilateur médical selon l'invention, lequel est également similaire à celui de Fig. 1, mais dans lequel le corps 2 du dispositif 1 d'aide au réglage a une forme (approximative) de demi-cercle et les première et seconde rangées d'emplacements 11, 21 disposés en arc de cercle, i.e. selon une courbe épousant le bord du demi-cercle.

Dans ce cas, les première et seconde séries d'emplacements 10, 20, i.e. les première et seconde rangées d'emplacements 11, 21, sont agencés sur les deux faces du corps 2, comme en Fig. 1 à Fig. 3 ; toutefois, elles pourraient être portées par une même surface 11 du corps principal 2, comme illustré en Fig. 4.

En outre, l'élément-curseur 5 portant les éléments-indicateurs 13, 23 (i.e. flèches ici) est ici mobile en pivotement, i.e. rotation, autour d'un axe de rotation XX.

La seconde rangée 20 d'emplacements 21 n'est pas visible car portée par la deuxième surface 4 du corps principal 2. Elle est disposée de manière analogue à la première rangée 20 d'emplacements 21. De même, l'élément-curseur 5 porte un second élément-indicateur 23 se déplaçant en regard de la seconde rangée 20 d'emplacements 21, lorsque l'utilisateur déplace l'élément-curseur 5 pour positionner le premier élément-indicateur 13 en regard d'un des premiers emplacements 11, comme expliqué ci-avant.

A noter, que dans ce mode de réalisation, le curseur pivotant pourrait être remplacé par un curseur translatif guidé par rail, comme sur Fig. 4, ou un autre système analogue.

D'une façon générale, l'une et/ou l'autre des surfaces 3, 4 du corps principal 2, peuvent comprendre d'autres marquages, graphiques, dessins ou indications, notamment en couleurs.

## Revendications

1. Dispositif (1) d'aide au réglage d'un ventilateur médical servant à fournir un gaz respiratoire à un patient comprenant :
- un corps principal (2) comprenant une première surface (3) et une seconde surface (4) opposée à la première surface (3),
- une première série d'emplacements (10) comprenant plusieurs emplacements (11) juxtaposés les uns aux autres, agencée sur la première surface (3), chaque emplacement (11) comprenant une indication (12) relative au ressenti du patient par rapport à une ventilation lui ayant été appliquée,
- une seconde série d'emplacements (20) comprenant plusieurs emplacements (21) juxtaposés les uns aux autres, agencée sur la première surface (3) ou la seconde surface (4), chaque emplacement (21) comprenant au moins une indication de réglage du ventilateur (22),
- un premier élément indicateur (13) agencé en regard de la première série d'emplacements (10) et mobile relativement aux emplacements (11) de la première série d'emplacements (10), et
- un second élément indicateur (23) agencé en regard de la seconde série d'emplacements (20) et mobile relativement aux emplacements (21) de la seconde série (20) d'emplacements (21),
et dans lequel le premier élément indicateur (13) coopère avec le second élément indicateur (23) de sorte qu'un positionnement par l'utilisateur du premier élément indicateur (13) face à l'un des emplacements (11) de la première série d'emplacements (10) engendre un déplacement simultané du second élément indicateur (23) et un positionnement dudit second élément indicateur (23) face à l'un des emplacements (21) de la seconde série (20) d'emplacements (20).

2. Dispositif selon la revendication 1, **caractérisé en ce que** le corps principal (2) a une forme plane, en particulier une forme rectangulaire.

3. Dispositif selon la revendication 1, **caractérisé en ce que** la seconde série d'emplacements (20) est agencée sur la seconde surface (4) et le second élément indicateur (23) agencé mobile en regard de la seconde surface (4).

4. Dispositif selon la revendication 1, **caractérisé en ce que** le premier élément indicateur (13) et le second élément indicateur (23) sont agencés sur un élément-curseur (5) commun mobile.

5. Dispositif selon la revendication 4, **caractérisé en ce que** l'élément-curseur (5) comprend un manchon (6) agencé coulissant autour du corps principal (2).

6. Dispositif selon la revendication 5, **caractérisé en ce que** le corps principal (2) comprend des butées d'extrémités (7) pour stopper tout coulissement du manchon (6).

7. Dispositif selon la revendication 1, **caractérisé en ce que** la première série d'emplacements (10) comprenant des emplacements (11) agencés en une première rangée et/ou la seconde série d'emplacements (20) comprenant des emplacements (21) agencés en une seconde rangée.

8. Dispositif selon la revendication 1, **caractérisé en ce que** le corps principal (2) est en carton, papier ou polymère.

9. Dispositif selon l'une des revendications 1 ou 4, **caractérisé en ce que** le premier élément indicateur (13) et le second élément indicateur (23) comprennent un marquage ou repère, ou une fenêtre de lecture.

10. Dispositif selon la revendication 1, **caractérisé en ce que** le premier élément indicateur (13) et le second élément indicateur (23) sont mobiles simultanément en translation ou en pivotement, de préférence en translation.
